Europäisches Patentamt

European Patent Office

Office européen des brevets

(19)

(11) Publication number: **0 414 921 A1**

## EUROPEAN PATENT APPLICATION
### published in accordance with Art. 158(3) EPC

(12)

(21) Application number: 90904674.0

(22) Date of filing: 19.03.90

(86) International application number:
PCT/JP90/00367

(87) International publication number:
WO 90/11350 (04.10.90 90/23)

(51) Int. Cl.5: **C12N 5/24, C12P 21/08, A61K 39/395**

(30) Priority: 20.03.89 JP 66326/89
20.03.89 JP 66327/89
20.03.89 JP 66328/89
20.03.89 JP 66329/89
11.05.89 JP 116048/89

(43) Date of publication of application:
06.03.91 Bulletin 91/10

(84) Designated Contracting States:
CH DE DK ES FR GB IT LI NL SE

(71) Applicant: MITSUI TOATSU CHEMICALS, Inc.
2-5 Kasumigaseki 3-chome
Chiyoda-Ku Tokyo 100(JP)

(72) Inventor: FUKUDA, Tamotsu 2142, Tougo
Mobara-shi
Chiba-ken 297(JP)
Inventor: ONO, Yasushi 13-4, Honcho
2-chome
Shiki-shi
Saitama-ken 353(JP)
Inventor: SHIGETA, Shiro 147-28,
Omori-aza-Kubouchi

Fukushima-shi
Fukusima-ken 960-11(JP)
Inventor: KUROIWA, Yasuyuki 2791-1, Mutuno
Mobara-shi
Chiba-ken 297(JP)
Inventor: OOKA, Hisayoshi Rezion Ogata 207
16, Gounome-aza-Horai-chou Fukushima-shi
Fukusima-den 960(JP)
Inventor: TAKAGI, Shiro 2142, Tougo
Mobara-shi
Chiba-ken 297(JP)
Inventor: OKUYA, Hiroaki 2142, Tougo
Mobara-shi
Chiba-ken 297(JP)
Inventor: KONO, Naoko
90-1, Machibo Mobara-shi
Chiba-ken 297(JP)
Inventor: YANAI, Yuko
90-1, Machibo Mobara-shi
Chiba-ken 297(JP)

(74) Representative: Harvey, David Gareth et al
Graham Watt & Co. Riverhead
Sevenoaks Kent TN13 2BN(GB)

(54) HUMAN MONOCLONAL ANTIBODY REACTIVE WITH PSEUDOMONAS AERUGINOSA, CELL WHICH PRODUCES THE ANTIBODY, METHOD OF PRODUCTION, AND PHARMACEUTICAL PREPARATION.

(57) A new human-human hybridoma which can secrete large amounts of human monoclonal antibodies which are reactive with at least one serotype of principal causative bacteria of Pseudomonas aeruginosa infections in a serum-free medium. Pharmaccutical preparations comprising various combinations of the obtained antibodies are excellent in the effect of preventing or treating Pseudomonas aeruginosa infections.

# HUMAN MONOCLONAL ANTIBODIES HAVING REACTIVITY WITH PSEUDOMONAS AERUGINOSA, CELLS CAPABLE OF PRODUCING THE SAME, METHODS FOR PRODUCTION THEREOF AND PHARMACEUTICAL PREPARATIONS THEREOF

## FIELD OF THE INVENTION

The present invention relates to a human-human hybridoma cell line which can stably supply in large quantity human monoclonal antibodies having reactivity with at least one serotype bacteria of the major causative bacteria of infectious diseases with Pseudomonas aeruginosa. The present invention also relates to human monoclonal antibodies produced by the human-human hybridoma cell line and to pharmaceutical compositions for prophylaxis and treatment of Pseudomonas aeruginosa infectious diseases using the human monoclonal antibodies as an effective ingredient.

## PRIOR ART

Pseudomonas aeruginosa infectious diseases are opportunistic infectious diseases frequently caused in patients with various basal diseases or patients who have been given immunosuppressive drugs. Pseudomonas aeruginosa infectious diseases are currently considered to be infectious diseases which are most difficult to treat. That is, Pseudomonas aeruginosa not only shows resistance to almost all antibiotics conventionally used, but also has a strong tendency to readily induce resistance to antibiotics developed in recent years. For this reason, investigations have been made on both prophylaxis and therapy, to enhance the ability of the host to kill Pseudomonas aeruginosa.

In recent years, immune serum globulins having as the effective ingredient human immunoglobulin purified from healthy donors' sera or plasma, or its chemically modified derivative have often been used for the treatment of Pseudomonas aeruginosa infectious diseases. However, among the antibodies contained in these immune serum globulins, the amount of antibody having affinity to Pseudomonas aeruginosa and effective for the treatment desired is not certain or its content is small Thus, the prophylactic and therapeutic effects of these immune serum globulins are often problematic. There is thus a need to develop human monoclonal antibodies effective in small doses for this purpose.

Pseudomonas aeruginosa is classified by its serotype, using an immune antibody capable of recognizing the O-polysaccharide side chain on the lipopolysaccharide (hereafter simply referred to as LPS) present on the outer membrane, i.e., an antibody to Pseudomonas aeruginosa serotype-specific O-antigen The serotype classification of Pseudomonas aeruginosa is currently a topic of discussion. In Japan, serologic classification of the Serotyping Comittee for the Japan Pseudomonas aeruginosa Society [Homma, Japan J. Exp. Med., 46, 329-336 (1976) ] has been widely used which classifies Pseudomonas aeruginosa into 13 serotypes from group A to group M. Each serotype of Pseudomonas aeruginosa is isolated from patients with Pseudomonas aeruginosa infectious diseases in the clinics with a constant ratio. It is known that a ratio of 5 serotypes of groups A, B, E, G and I is predominant in the 13 serotypes.

On the other hand, mouse monoclonal antibody to Pseudomonas aeruginosa has been produced by the mouse-mouse hybridoma technique developed by Köhler and Milstein [Köhler and Milstein, Nature, 256 , 495-497 (1975)]. Since then [for example, Hancock et al., Infect. Immun., 37 , 166-171 (1982)], the technique has been applied to serotype classification for example, Meiji Seika, EP 101039] or used for basic research for a survey of monoclonal antibodies useful for protection from infections, etc.

Sadoff et al. reports that mouse monoclonal antibody to the O-polysaccharide side chain on the serotype-specific LPS molecule of Pseudomonas aeruginosa has a high protection activity against lethal challenge with bacteria of the corresponding serotype in the experimental infection using mice [Sadoff et al., Abstracts of the 1982 Interscience Conference on Antimicrobial Agents and Chemotherapy, No. 253 (1982)]. In the following reports, the effectiveness of mouse or human monoclonal antibodies to serotype-specific O-antigen of Pseudomonas aeruginosa is shown in in vivo or in vitro tests [for example, Sawada et al., J. Infect. Dis., 150, 570-576 (1984); Yoshiaki Nakamura et al., Japanese Journal of Bacteriology, 39, 337 (1984); Pennington, Infect. Immun., 54 , 239-244 (1986); Suzuki et al., Microbiol. Immunol, 31 , 959-966 (1987), Zwerrnik et al., Infect. Immun., 56 , 1873-1879 (1988)]. Furthermore, application of serotype-specific human monoclonal antibodies to prophylaxis and therapy of Pseudomonas aeruginosa infectious diseases has been described in a patent application directed to antibodies capable of independently recognizing the O-polysaccharide side chain on the serotype-specific LPS molecule [Japanese Patent Application Laid-Open No. 60-248626] and a patent application directed to antibodies capable of recognizing a plurality of O-polysaccharide side chains in common [International Patent Application Laid-Open No. WO 88/04669], by the present inventors as well as in other patent applications [Genentech Systems Corporation, EP 163493 and BE 905890; Teijin Limited, WO 86/03754; Wakunaga Pharmaceutical Co., Ltd., Japanese Patent Application Laid-Open No. 61-091134; Merck & Company Incorporated, EP 256713].

PROBLEMS SOLVED BY THE INVENTION

In general, human monoclonal antibody is produced by infecting human B cells with Epstein-Barr virus (hereafter simply referred to as EB virus) to convert the B cells into EB virus transformed cells or by fusing human antibody producing cells such as B cells and a parent cell line capable of infinite proliferation to convert it into a human-mouse hetero hybridoma or human-human hybridoma.

In the transformant produced by EB virus transformation, the amount of antibodies produced is generally poor and subculture stability is inferior. Furthermore, due to relatively high auxotrophy, the transformant is not suited for mass production using a serum-free medium. In the case of fusing with human antibody producing cells using mouse myeloma as the parent cell line, the produced human-mouse hetero hybridoma synthesizes and secretes mouse protein together with human antibodies and hence, it is not always suitable to use the transformant as the cell line for producing human monoclonal antibody administered to a human. Further, in the case of producing human-human hybridoma by fusion with human antibody producing cells using cells having a human chromosome alone and capable of infinite proliferation as the parent cell line, some problems are encountered. For example, the fusion efficiency of a human myeloma-derived parent cell line with human antibody producing cells is poor. Although the fusion efficiency of EB virus transformed cells-derived parent cell line with human antibody producing cells is relatively high, most of the resulting human-human hybridomas also produce antibodies having no antigen specificity and at the same time, produce only a minor amount of antibodies. The human-human hybridomas produced by fusion of human antibody producing cells and the parent cells derived from the hybridoma between human myeloma cells and EB virus transformed cells produce a relatively high amount of antibodies, but the possibility of simultaneously producing antibodies having no antigen specificity is not eliminated.

MEANS FOR SOLVING THE PROBLEMS

The present inventors have found human-human hybridomas capable of producing human monoclonal antibodies having reactivity with at least one serotype bacteria which are the major causative bacteria of Pseudomonas aeruginosa infections; that the human-human hybridomas can be stably proliferated in various media and can maintain a relatively large quantity of antibody production over long periods of time; and further that these human-human hybridomas are cultured and human monoclonal antibodies having reactivity with at least one serotype bacteria which are the major causative bacteria of Pseudomonas aeruginosa infections can be prepared from the culture.

Based on these results, the present inventors have examined the protective activity of these antibodies produced by the human-human hybridomas against Pseudomonas aeruginosa infections and have come to accomplish the present invention.

The term "the major causative bacteria" as used in this application refers to bacteria of 5 serotypes of groups A, B, E, G and I in the serologic classification by the Serotyping Comittee for the Japan Pseudomonas aeruginosa Society.

The term "human-human hybridoma" as used in this application refers to hybridomas having a human chromosome alone produced by fusing the parent cell line capable of infinite proliferation and having a human chromosome alone with human antibody producing cells.

The term "selection characteristic" as used herein refers to the chemical or physical properties of the parent cell line which enables to select the produced hybridoma from the unfused cells. For example, in the case of using a parent cell line resistant to 8-azaguanine or 6-thioguanine and ouabain as the selection characteristic, only the hybridoma derived from the human antibody producing EB virus transformed cells can survive in the medium containing hypoxanthine, azaserine and ouabain.

A parent cell line having the selection characteristic described above can be appropriately selected. Furthermore, the human antibody producing cells capable of reacting with Pseudomonas aeruginosa can be appropriately selected from human B cells and cells derived therefrom.

Hereafter the present invention is described by referring to the case of producing a human-human hybridoma using as the parent cell line having a human chromosome alone and infinite growth ability and resistance to 8-azaguanine and ouabain and using as the human antibody producing cells human antibody producing EB virus transformed cells having reactivities respectively with groups A, B, E, G and I of Pseudomonas aeruginosa in the serologic classification by the Serotyping Comittee for the Japan Pseudomonas aeruginosa Society, through fusion between both cells.

1. Pseudomonas aeruginosa used:

For convenience, the classification of Pseudomonas aeruginosa used in the present invention follows the serologic classification by the Serotyping Comittee for the Japan Pseudomonas aeruginosa Society as stated above. In the present invention, strains belonging to group A to group M are used.

Strains belonging to group A to group M can be acquired from the American Type Culture Collection (ATCC) and the Medical and Science Research Institute of Tokyo University. 2. Production of human-human hybridoma:

Human-human hybridomas capable of producing human monoclonal antibodies having reactivities respectively with groups A, B, E, G and I of Pseudomonas aeruginosa in the serologic classification by the Serotyping Comittee for the Japan Pseudomonas aeruginosa Society can be produced by fusing cells of the parent cell line MP 4109 or its subcultured cell line for producing hybridoma with human antibody producing cells, in a manner similar to a known method [textbook "MONOCLONAL ANTIBODIES", page 363, published by Plenum Press (1980) and the like]. As the human antibody producing cells, there may be used B cells obtained from the peripheral blood, lymph node, tonsil or spleen of healthy donors observed to be able to produce antibodies to Pseudomonas aeruginosa or patients with a past history of Pseudomonas aeruginosa infectious diseases or, from cord blood upon delivery, etc., by known methods. However, it is preferred to use the colonies of EB virus transformant obtained by infecting B cells with EB virus to perform transformation, culturing the transformant for a definite period of time, secreting antibodies having reactivity with Pseudomonas aeruginosa in the culture supernatant, and selecting colonies of EB virus transformed cells in which the secreted antibodies are detected or by using the cell line singly selected from these colonies of EB virus transformed cells.

Next, each step is now described in detail.

Isolation and concentration of B cells from blood or tissues described above can be efficiently carried out by the specific gravity centrifugation method using cell separation fluid such as Ficoll-Conray (Registered mark) solution, etc., the E rosette formation method, the Panning method, etc. preferably in combination. In addition, after B cells are cultured in the medium supplemented with pokeweed mitogen (PWM) for several days to grow B cells, the proliferated B cells may also be provided for the cell fusion.

Transformation of B cells with EB virus can be carried out in a manner similar to known methods [for example, Steinitz et al., Nature, 269, 420-422 (1977)]. B95-8 cells (cells derived from marmoset leucocyte derived cells capable of producing infectious EB virus) are cultured in RPMI 1640 medium containing 20% fetal calf serum (hereafter simply referred to as FCS), the RPMI 1640 medium containing FCS being sometimes simply referred to as the medium, and the culture supernatant obtained by centrifugation on Day 7 close to the stationary state is used as virus solution [Ono et al., Proceedings of the Japanese Society for Immunology, 4, 399-401 (1974)]. B cells are centrifuged and the supernatant is removed by suction. The virus solution is added to the resulting pellets to disperse the pellets followed by incubation at 37° C for 30 minutes to an hour in the presence of 5% carbon dioxide gas. After the incubation, centrifugation is performed and the supernatant is removed by suction. Then, the medium is added to the pellets at a cell density of $1 \times 10^5$/ml to $5 \times 10^5$/ml to disperse the cells. The cell dispersion is dispensed in each well of a 24 well culture plate or a 96 well culture plate followed by incubation at 37°c for 2 to 4 weeks in the presence of 5% carbon dioxide gas. During the period, it is preferred that half of the medium is exchanged by fresh medium at 3 or 4 day intervals.

Detection of the antibody having reactivity with Pseudomonas aeruginosa can be made by ordinary radioimmunoassay, enzyme-linked immunosorbent assay (hereafter simply referred to as ELISA), etc. [ "MONOCLONAL ANTIBODIES", page 144, published by Kodansha Publishing Co. (1983), etc.]. In the present invention, ELISA is used. That is, the dot immunobinding assay (hereafter simply referred to as DIBA) which comprises fixing Pseudomonas aeruginosa previously treated with 0.3% formalin onto a membrane filter, reacting it with the cell culture supernatant in a vessel for a definite period of time, then reacting it with enzyme conjugated rabbit anti-human antibody and determining the presence or absence of the production of the desired antibody and its amount produced by coloration of the substrate by the enzyme reaction [Anal. Biochem., 119 , 142-147 (1982)] is used as a handy assay method.

The well in which the desired antibody is present is selected by the ELISA described above by observing the culture supernatant of each well in which growth colonies of EB virus transformed cells are observed. Then, the cells in the well are subjected to cloning by the soft agar method ["ADVANCED TISSUE CULTURES FOR IN VITRO ASSAY AND PRODUCTION", page 289, published by Soft Science Inc. (1985), etc.] or by the limiting dilution method ["MONOCLONAL ANTIBODIES", page 73, published by Kodansha Publishing Co. (1983), etc.]. After growth of the cells is further observed by cloning, an assay is again performed by ELISA. By cloning one or more times, a single cell line capable of secreting the desired antibody alone can be obtained.

Fusion of MP 4109 and human antibody producing cells can be effected using conventional reagents for fusion such as polyethylene glycol (hereafter simply referred to as PEG) or using virus particles or virions such as Sendai virus (hemagglucinating virus of Japan: HVJ), etc. For example, RPMI 1640 medium or Dulbecco's modified Eagle's medium (DMEM) supplemented with PEG having a mean molecular weight of about 1000 to about 6000 in a concentration of 30% to 50% (W/V) is preferred as a fusing agent. Further, in order to enhance a fusion efficiency, it is preferred to supplement the fusing agent with dimethylsulfoxide (DMSO). Furthermore, physical means using an apparatus for electric fusion, etc. may be also applicable to enhancing a fusion efficiency.

For example, MP 4109 and cells in the well in which production of the desired antibody was noted after transformation with EB virus or antibody producing cells isolated from peripheral blood, etc. are mixed in a ratio of approximately 1 : 1 to 1 : 10 and a medium for cell fusion (RPMI 1640 medium containing 50% PEG and 10% DMSO, or the like) is added to the mixture to fuse the cells. Next, the cells are dispersed in a medium suited for growth of the fused hybridoma alone (hereafter simply referred to as selective medium) at a cell density of $1 \times 10^5$/ml to $5 \times 10^6$/ml. The cell dispersion is separately charged in a 24 well or 96 well culture plate followed by incubation at 37°C for 2 to 4 weeks in the presence of 5% carbon dioxide gas. During the period, it is preferred that half of the medium is exchanged by fresh selective medium at 3 to 5 day intervals. In this case, the copresence of mouse peritoneal exudate cells, etc. as feeder cells can accelerate growth of the hybridoma. In the case that the human antibody producing cells are incapable of infinite growth (B cells), a medium containing hypoxanthine, aminoputerine and thymidine (hereafter simply referred to as HAT medium) or a medium containing hypoxanthine and azaserine (hereafter simply referred to as HA medium) can be used as the selective medium. Further, if the human antibody producing cells are cells capable of infinite growth such as EB virus transformed cells, etc., a medium obtained by adding ouabain to HAT medium (NAT-O medium) or a medium obtained by adding ouabain to HA medium (HA-O medium) can be used as the selective medium. The culture supernatant of each well in which growth colonies of the hybridoma are observed is examined, and the well in which the desired antibody is present is selected by ELISA described above followed by cloning by the limiting dilution method. After the cell growth is observed by cloning, assay is again performed by ELISA. By cloning one or more times, the single cell line capable of secreting the desired antibody alone can be obtained.

The human-human hybridoma of the present invention can be cultured in a conventional medium. For example, the human-human hybridoma is dispersed in a medium at a cell density of $5 \times 10^4$/ml to $2 \times 10^6$/ml and the dispersion is inoculated on a suitable cell culture vessel followed by culturing at 37°C in the presence of 5% carbon dioxide gas. As the medium, basal medium such as RPMI 1640 medium, DMEM, etc. supplemented with a suitable amount of FCS is preferred. In addition, various low serum content or serum-free media can also be appropriately used. For example, NYSF 404 serum-free medium alone or NYSF 404 serum-free medium supplemented with a suitable amount of bovine serum albumin is preferred. Subculture may be performed by repeating the procedures of recovering the cells and inoculation in intervals of 3 to 7 days.

The human-human hybridoma of the present invention can be stored by freeaing in a conventional manner. For example, the cells are dispersed in a suitable storage solution for freezing at a cell density of $1 \times 10^5$/ml to $5 \times 10^7$/ml. The dispersion can be stored by freezing in liquid nitrogen or over liquid nitrogen, or in a refrigerator at -20 to -80°C. It is preferred to use the cell freeze storage solution by appropriately adding animal serum, albumin, methyl cellulose, glucose, dimethylsulfoxide, etc. to the basal medium described above or to a neutral buffer solution, etc.

The frozen cells can be thawed in a conventional manner. For example, the storage solution containing the frozen cells is rapidly thawed in warm water, the cells are washed with a medium, etc. to wash away DMSO contained in the storage solution and the cells are then dispersed in the medium followed by incubation.

The amount of immunoglobulin in the culture supernatant can be assayed by conventional ELISA. For example, in the case of using ELISA, the amount of immunoglobulin in the culture supernatant can be determined by immobilizing anti-human immunoglobulin antibody onto a solid phase (the antibody used in this case is hereafter simply referred to as fixed antibody), reacting a part of the culture supernatant with it, then reacting with enzyme conjugated anti-human immunoglobulin antibody, adding substrate to form a color in response to the enzyme reaction, and measuring the degree of coloration. Human IgM can be determined using anti-human IgM (mu chain specific) antibody as the fixed antibody and peroxidase-labeled anti-human IgM (mu chain specific) antibody as the enzyme conjugated antibody. 3. Production of human monoclonal antibody:

The human-human hybridoma of the present invention is capable of synthesizing and secreting the heavy chain derived from the antibody producing cell line and can be stably subcultured and proliferated in any ordinary medium used for the cultivation of animal cells over long periods of time. The human-human hybridoma is also capable of producing antibodies even in a serum-free medium which is free from the danger that unknown impurities derived from medium might be intermingled upon purification of the antibodies from the culture. The human-human hybridoma is thus most suited for obtaining human monoclonal antibody as the raw material for preparing compositions for preventing and treating Pseudomonas aeruginosa infectious diseases.

After culturing the human-human hybridoma of the present invention is serum-free medium, the resulting human monoclonal antibodies having reactivities respectively with groups A, B, E, G and I of Pseudomonas aeruginosa in the serologic classification by the Serotyping Comittee for the Japan Pseudomonas aeruginosa Society, can be relatively easily purified from the culture to a high purity by optional and conventional technique, for example, physicochemical purification such as gel filtration, ion exchange chromatography, adsorption chromatography using hydroxyapatite, etc.; affinity chromatography using a carrier to which antigen or a substance having affinity to human monoclonal antibody (e.g., protein A, anti-human immunoglobulin antibody, etc.) has been fixed, electrophoresis, precipitation such as the ammonium sulfate precipitation method, etc.; individually or in combination.

4. Production of a composition comprising human monoclonal antibody:

The human monoclonal antibody of the present invention having reactivities respectively with groups A, B, E, G and I of Pseudomonas aeruginosa in the serologic classification by the Serotyping Comittee for the Japan Pseudomonas aeruginosa Society has a high protective activity against the corresponding serotype Pseudomonas aeruginosa infectious diseases. The human monoclonal antibody of the present invention can be provided for prophylaxis and therapy of Pseudomonas aeruginosa infectious diseases, by itself or in the form of a liquid composition or freeze dried preparation with additives, excipients, etc. conventionally used. In general, the additives and excipients are appropriately chosen from naturally occurring products and compounds used in biological preparations. In order to maintain stability of the antibody, animal protein such as albumin, etc., polysaccharides such as dextran, etc., amino acids and carbohydrates give good results. Furthermore, the human monoclonal antibody of the present invention may also be mixed with other monoclonal antibodies or polyclonal antibodies reactive with Pseudomonas aeruginosa or to microorganisms other than Pseudomonas aeruginosa to produce pharmaceutical compostions.

5. Prophylaxis and therapy of infectious diseases by human monoclonal antibody:

In prophylaxis and therapy of actual Pseudomonas aeruginosa infectious diseases, the composition comprising the human monoclonal antibody of the present invention may be administered singly or as admixture of two or more human monoclonal antibodies. Alternatively, the composition may also be mixed with other monoclonal antibodies reactive with Pseudomonas aeruginosa or, with compositions comprising the same or with immune serum globulins.

The human monoclonal antibody of the present invention having reactivities with one or more groups A, B, E, G and I of Pseudomonas aeruginosa in the serologic classification by the Serotyping Comittee for the Japan Pseudomonas aeruginosa Society or the composition comprising the human monoclonal antibody can be administered directly to humans for prophylaxis and therapy of Pseudomonas aeruginosa infectious diseases. Dosages and administration routes are appropriately chosen but a preferred dose is in the range of from 0.01 to 10 mg/body weight (kg). The administration route may be suitably chosen from intracutaneous, subcutanous, intramuscular, intravenous and the like routes of administration.

The present invention provides a human monoclonal antibody producing cell line for industrial production which can be applied over wide fields such as prophylaxis, therapy, diagnosis and the like of Pseudomonas aeruginosa infectious diseases.

The present invention is further described in more detail by referring to the examples that follow but is not deeded to be limited thereto.

The parent cell line MP 4109 used for producing hybridoma has been deposited in the Fermentation Research Institute of the Agency of Industrial Science and Technology of Japan under accession No. FERM BP-2129 on October 27, 1988. The human monoclonal IgM producing EB virus transformed cell line MP 5046, which is cross-reactive with groups A and F of Pseudomonas aeruginosa ,has been deposited in the Fermentation Research Institute of the Agency of Industrial Science and Technology of Japan under accession No. FERM BP-1599 on December 9, 1987. The human monoclonal IgM producing EB virus transformed cell line MP 5038,which is cross-reactive with groups E and F of Pseudomonas aeruginosa, has been deposited in the Fermentation Research Institute of the Agency of Industrial Science and Technology of Japan under accession No. FERM BP-1596 on December 7, 1987. The human monoclonal IgM producing EB virus transformed cell line MP 5050,which is cross-reactive with groups G and H of Pseudomonas aeruginosa, has been deposited in the Fermentation Research Institute of the Agency of Industrial Science and Technology of Japan under accession No. FERM BP-1600 on December 9, 1987. The human monoclonal IgM producing EB virus transformed cell line MP 5035, which is cross-reactive with groups I and D of Pseudomonas aeruginosa, has been deposited in the Fermentation Research Institute of the Agency of Industrial Science and Technology of Japan under accession No. FERM BP-1598 on December 9, 1987. As the standard serotype Pseudomonas aeruginosa in the serologic classification by the Serotyping Comittee for the Japan Pseudomonas aeruginosa Society, ATCC 27577 (IID 1001) was used for group A of Pseudomonas aeruginosa; ATCC 27578 (IID 1002), ATCC 27583 (IID 1007), ATCC 27589 (IID, 1013) and IID 5004 for group B of Pseudomonas aeruginosa; ATCC 27580 (IID 1004) for group D of Pseudomonas aeruginosa; ATCC 27581 (IID 1005) for group E of Pseudomonas aeruginosa; ATCC 27582 (IID 1006) for group F of Pseudomonas aeruginosa; ATCC 27584 (IID 1008) for group G of Pseudomonas aeruginosa; ATCC 27585 (IID 1009) for group H of Pseudomonas aeruginosa; and ATCC 27586 (IID 1010) for group I of Pseudomonas aeruginosa.

Example 1 1. Production of a hybridoma capable of producing monoclonal antibody to Pseudomonas aeruginosa:

(1) Cell fusion:

MP 4109 and MP 5046 were grown in RPMI 1640 medium containing 10% FCS (hereafter sometimes simply referred to as 10% FCS medium) and then collected and washed with RPMI 1640 medium, respectively. The cells of $2 \times 10^7$ each were mixed with each other in a plastic centrifuge tube of a 50 ml volume. After centrifugation (175 x g, 10 minutes), the supernatant was removed by suction and, 0.5 ml of RPMI 1640 medium containing 50% PEG (M.W.:1500, Wako Chemicals) and 10% DMSO was gently added directly to the cell pellets. While slowly rotating, the cells were fused. Two minutes after, 10 ml of RPMI 1640 medium was added to the system. After gently agitating, centrifugation (175 x g, 10 minutes) followed. After the supernatant was removed by suction, RPMI 1640 medium containing 20% FCS, $2 \times 10^{-4}$ M of hypoxanthine (Sigma), 1 $\mu$g/ml azaserine (Sigma) and $5 \times 10^{-6}$ M of ouabain (Sigma) (hereafter sometimes simply referred to as HA-O medium) was added to the cell pellets followed by suspending at a cell density of $1 \times 10^6$/ml. Then, the suspension was inoculated on a 96 well flat bottom culture plate in 0.1 ml each per well (384 wells in total). The cells were subjected to stationary culture at 37°C in the presence of 5% carbon dioxide gas. Four days after, 0.1 ml of HA-O medium was added and thereafter, half of the volume of HA-O medium was replaced by fresh HA-O medium at 3 to 5 day intervals. Four to five weeks after, cell growth was observed in 65 wells in total.

(2) Detection of antibody to Pseudomonas aeruginosa :

The presence or absence of human antibody to group A Pseudomonas aeruginosa in the culture supernatant was examined by the DIBA method. The culture supernatant, 0.1 ml, in each well was reacted with a nitrocellulose membrane filter (3.1 mm square; Toyo Roshi) with grid to which 0.4 $\mu$g/dot of group A Pseudomonas aeruginosa ATCC 27577 treated with formalin and then dried had been fixed, in a 96 well U-shaped bottom microplate. After reacting at room temperature for 2 hours, the filter was reacted with peroxidase-labeled rabbit anti-human immuno-globulin antibody (Dako Co.) for 2 hours and a color was formed using 4-chloro-1-naphthol as a substrate. When a color was observed on the nitrocellulose membrane filter by the naked eye, antibody production was judged to be positive. (3) Cloning:

Among the culture supernatant 65 wells in which cell growth was observed, the production of human antibody to group A Pseudomonas aeruginosa was observed in 51 wells of the culture supernatant. From 51 wells, 18 wells containing the cells well grown were selected and the cells in the wells were collected, respectively, and accurately counted using a hemocytometer. The cells were dispersed in HA-O medium to prepare a cell suspension at a cell density of 20/ml. After the supernatant in each well of a 96 well flat bottom culture plate, on which 1 x 10⁵/well of mouse spleen cells had previously been inoculated(hereafter simply referred to as feeder plate), was removed, the cell suspension was inoculated on the feeder plate by 0.1 ml each per well followed by stationary culture at 37° C in the presence of 5% carbon dioxide gas. One feeder plate was used per each cell. Four days after, 0.1 ml of HA-O medium was added and then, half of the volume of HA-O medium was replaced by fresh HA-O medium at 3 to 5 day intervals. The presence or absence of human antibody to group A Pseudomonas aeruginosa in the culture supernatant was examined by the DIBA method in the wells in which cell growth was observed two to four weeks after. The cells in the well in which production of the antibody reactive with group A Pseudomonas aeruginosa was again subjected to cloning as described above. By cloning twice, 12 strains of hybridomas from MP 5120 to MP 5131 producing the human monoclonal antibody capable of cross-reacting with groups A and F Pseudomonas aeruginosa in the serologic classification by the Serotyping Comittee for the Japan Pseudomonas aeruginosa Society were obtained.

MP 5121 was deposited in the Fermentation Research Institute of the Agency of Industrial Science and Technology of Japan under accession No. FERN BP-2270.

The hybridomas sufficiently grown in the 96 well flat bottom culture plate were cultured gradually on a large scale. After the cells were suspended in a cell storage solution composed of 15% RPMI 1640 medium, 75% FCS and 10% DMSO at a density of 1 x 10⁶/ml, the suspension was separately charged in a 2 ml freezing tube. After cooling it to -20° C at a rate of 1° C/min, the tube was stored freezingly in liquid nitrogen.

(4) Determination of amount of antibody produced:

The amount of IgM which the hybridomas secreted outside the cells was determined as follows. The cells in the logarithmic phase were collected and suspended in 10% FCS medium at a density of 1 x 10⁶/ml, and 1 ml each of the suspension was inoculated on each well of a 6 well culture plate followed by stationary culture at 37° C in the presence of 5% carbon dioxide gas. Twenty four hours after, the culture supernatant wad separated by centrifugation (250 x 9, 10 minutes) and an amount of IgM in the supernatant was quantitatively determined by ELISA. In the 12 hybridomas, 10⁶ cells secreted 6 to 56 μg of human IgM to the culture supernatant for 24 hours.

(5) Determination of stability of the cell line in continuous subculture:

Stability of the cell line in continuous subculture was examined in terms of cell growth efficiency and antibody productivity.

Stability of the growth was determined by measuring growth curves of the cells at the time of initiating culture and 3 months after the initiation of continuous subculture. Stability of antibody production was examined by measuring amounts of IgM antibody produced at the time when the culture started, and one month, 2 months and 3 months after the initiation of culture by ELISA as in (4) above.

Continuous subculture was carried out as follows. The cells were independently suspended in duplicate in 10% FCS medium at a cell density of 5 x 10⁴/ml. Then 4 ml of each suspension was inoculated on a cell culture flask having a bottom area of 25 cm² followed by stationary culture at 37° C in the presence of 5% carbon dioxide gas. The cells were collected at 3 or 4 day intervals and again suspended in fresh 10% FCS medium at the same density followed by stationary culture. The procedure was carried out continuosly for 3 months.

Growth curve was measured as follows. The cells in the logarithmic phase which had been independently cultured in duplicate were collected and suspended at a density of 5 x 10⁴/ml in 10% FCS medium. Then 1 ml of the suspension was inoculated on each well of a 6 well culture plate (6 wells in total) followed by stationary culture at 37° C in the presence of 5% carbon dioxide gas. The culture in one well was collected every day to count viable cell and dead cell densities. The amount of IgM in the culture supernatant was determined by ELISA.

MP 5121 showed almost the same growth curve with the cells at the beginning of cultivation and 3 months after the cultivation. The doubling time calculated from the growth curve was 25.5 hours. Furthermore, IgM secreted by 10⁶ cells for 24 hours was 56 μg at the begining of cultivation, 40 μg one month after the initiation of cultivation, 45 μg 2 months after and 42 μg 3 months after, indicating that there was no significant difference in the amount of antibody produced during continuous subculture for 3 months.

Example 2 1. Culture of cells and purification of the antibody:

The frozen cells of MP 5121 were thawed and cultured in 10% FCS medium on a large scale. After culturing the cells in NYSF 404 medium [Noritsugu Yabe, TISSUE CULTURE, 11 , 458 (1985)] on a larger scale, the cells were collected and suspended in 500 ml of NYSF 404 medium at a cell density of $5 \times 10^4$ /ml. The cells were then inoculated on 10 flasks (bottom area of 175 cm²) followed by stationary culture at 37° C for 5 days in the presence of 5% carbon dioxide gas. From the culture, 480 ml of the supernatant was obtained by centrifugation (400 x g, 20 minutes) and filtered through a membrane filter having a pore size of 0.22 micron.

After 480 ml of saturated ammonium sulfate aqueous solution was added to the filtrate, the mixture was allowed to stand at 4° C. The next day, the mixture was centrifuged (10,000 x g, 30 minutes) and the precipitates were collected. The precipitates were dissolved in 5 ml of PBS(-) and sufficiently dialyzed against PBS(-) to give the crude IgM fraction.

For purification, a hydroxyapatite-packed column for high performance liquid chromatography was used and fractionation was carried out at a flow rate of 1 ml/min. HCA-Column (guard column; 4 mm x 10 mm, body column: 7.6 mm x 100 mm, Mitsui Toatsu Chemical Co., Ltd.) was previously equilibrated with 0.01 M sodium phosphate buffer (pH 7.0) containing 0.15 M sodium chloride (hereafter simply referred to as Solution A) and 2 ml of the crude IgM fraction was added thereto. The column was washed with Solution A for 10 minutes and further with a solution obtained by adding Solution A to 0.25 M sodium phosphate buffer (pH 7.5,hereafter silly referred to as Solution B) in 75 : 25 by volume for 15 minutes. Thereafter, linear density gradient elution of 25% to 100% in the ratio of Solution B was performed over 20 minutes. The IgM fraction eluted as the single peak was thoroughly dialyzed against PBS(-). From 480 ml of the culture supernatant, the solution containing 12.3 mg of IgM (N4-2) was obtained.

Example 3 2. Production of hybridoma capable of producing monoclonal antibody to Pseudomonas aeruginosa:

(1) Preparation of EB virus solution:

B95-8 cells which produced and released EB virus were dispersed in RPMI 1640 medium containing 20% FCS (hereafter sometimes simply referred to as 20% FCS medium) at a cell density of $3 \times 10^5$/ml followed by stationary culture at 37° C in the presence of 5% carbon dioxide gas. The culture supernatant on Day 7 close to the stationary state wee collected by centrifugation (800 x g, 10 minutes) and filtered through a membrane filter (Millipore) having a pore size of 0.45 micron to give EB virus solution.

(2) Preparation of human lymphocyte:

From healthy donor whose serum antibody activity to group A Pseudomonas aeruginosa was detected up to 1000-fold dilution by the DIBA method, 50 ml of heparinized peripheral blood was collected. An equal volume of RPMI 1640 medium was added to the blood to dilute it 2-fold. The dilution was overlaid on the half volume of Ficoll-Paque (Pharmacia) not to ruffle the interface, followed by centrifugation (400 x g, 30 minutes) at room temperature. After centrifugation, the interface was withdrawn using a Pasteur pipette and an equal volume of 20% FCS medium was added thereto followed by centrifugation (250 x g, 10 minutes) at room temperature. After the precipitated cells were suspended in 20% FCS medium, centrifugation was further repeated to give human lymphocyte pellets (cell number: $4 \times 10^7$).

(3) Transformation with EB virus:

To $4 \times 10^7$ of human antibody producing cells was added 40 ml of the virus solution prepared in (1) followed by incubation at 37° C for an hour. After the incubation, the cells were collected by centrifugation (250 x g, 10 minutes). The cells were dispersed in 20% FCS medium. After the dispersion was adjusted to a cell density of $5 \times 10^5$/ml, 0.1 ml each of the dispersion was inoculated on a 96 well flat bottom culture plate followed by stationary culture at 37° C in the presence of 5% carbon dioxide gas. Four days after, 0.1 ml of 20% FCS medium was added and half of the volume of the medium was replaced by a fresh medium at 3 to 5 day intervals. With respect to the wells where cell growth was observed, the presence or absence of human antibody to group A Pseudomonas aeruginosa in the culture supernatant was examined by the DIBA method in a manner similar to Example 1, (2). The cells in the wells in which antibody production was detected were cultured in a 24 well culture plate on an enlarged scale.

(4) Cloning:

The cells in the wells in which production of human antibody to group A Pseudomonas aeruginosa antibody was detected by the antibody detection method were transferred to a Petri dish of 6 cm in diameter. The cells grown in the Petri dish were frozen and then stored in liquid nitrogen as in Example 1, (3). The frozen tube containing the cells was withdrawn from liquid nitrogen and thawed while stirring in a warm bath at 37°C. The cells were then suspended in 10 ml of 20% FCS medium. The cells were collected by centrifugation (250 x g, 10 minutes) and dispersed in 2 ml of 20% FCS medium. The dispersion was inoculated on a Petri dish of 6 cm in diameter followed by stationary culture at 37°C for 3 days in the presence of 5% carbon dioxide gas. The grown cells were subjected to cloning once by the soft agar method. Firstly, after the cell number was accurately counted with a hemocytometer, a cell suspension at a cell density of $1 \times 10^6$/ml was prepared. To 30 ml of medium containing 0.3% agarose (SeaPlaque Agarose (Registered mark), FMC) was added 0.1 ml of this cell suspension for mixing. Next, 3 ml of a medium containing the cells and 0.3% agarose were separately charged and fixed in a Petri dish of 6 cm which had been separately charged and fixed with 4 ml of medium containing 0.5% agarose (10 plates per each cell). The 6 cm Petri dish separately charged with the cells were subjected to stationary culture at 37°C in the presence of 5% carbon dioxide gas. Three to five weeks after, the cells grew on soft agar and colonies were observed with the naked eye. Then, each colony was transferred to each well of a 96 well flat bottom culture plate, which had been previously charged separately with 0.1 ml of 20% FCS medium, using a Pasteur pipette. Two days after, 0.1 ml of 20% FCS medium was further added thereto and an additional 2 days after, with respect to the wells where cell growth was observed, the presence or absence of human monoclonal antibody to Pseudomonas aeruginosa in the culture supernatant was examined by the DIBA method. The cells in the wells in which antibody production was judged to be positive was cultured in a 24 well culture plate on an enlarged scale. Three days after, with respect to the wells of the 24 well culture plate, the presence or absence of human antibody to Pseudomonas aeruginosa in the culture supernatant was examined by the DIBA method. Among them, the cells in the wells in which the activity of human antibody to Pseudomonas aeruginosa was high in the culture supernatant were cultured in order on an enlarged scale to give EB virus transformed cell colony §965N5 capable of producing human IgM antibody to group A Pseudomonas aeruginosa.

(5) Cell fusion:

Using $2 \times 10^7$ of EB virus transformed cell colony §965N5 capable of producing human IgM antibody to group A Pseudomonas aeruginosa and MP 4109, respectively, cell fusion was performed in a manner similar to Example 1, (1). The fused cells were suspended in HA-O medium at a cell density of $1 \times 10^6$/ml and the suspension was inoculated on a 96 well flat bottom culture plate in 0.1 ml each per well (384 wells in total). The cells were subjected to stationary culture at 37°C in the presence of 5% carbon dioxide gas. Four days after, 0.1 ml of HA-O medium was added thereto and thereafter, half of the volume of HA-O medium was replaced by fresh HA-O medium at 4 or 5 day intervals. With respect to 35 wells in which the cells grew, the presence or absence of human antibody to group A Pseudomonas aeruginosa in the culture supernatant was examined by the DIBA method in a manner similar to Example 1, (2). The antibody production was detected in the culture supernatant of 2 wells of 1B5 and 3G12. The cells in the 2 wells were cloned in a manner similar to Example 1, (3), respectively, to give hybridoma MP 5136 capable of producing human monoclonal antibody reactive only with group A Pseudomonas aeruginosa.

MP 5136 was deposited in the Fermentation Research Institute of the Agency of Industrial Science and Technology of Japan under accession No. FERM BP-2271.

(6) Determination of amount of antibody produced:

The amount of antibody produced by MP 5136 was determined in a manner similar to Example 1, (4).

As the result, in hybridoma MP 5136, $10^6$ cells secreted 13 μg of human IgM in the culture supernatant for 24 hours.

(7) Determination of stability of cell line in continuous subculture:

The growth ability and the stability of amount of antibody production of MP 5136 cell line were determined in a manner similar to Example 1, (5).

MP 5136 showed almost the same growth curve with the cells at the beginning of cultivation and 3 months after the cultivation. The doubling time calculated from the growth curve was 27 hours. Furthermore, IgM secreted by $10^6$ cells for 24 hours was 13 μg at the beginning of cultivation, 14 μg one month after the initiation of cultivation, 8 μg 2 months after and 12 μg 3 months after, indicating that there was no significant difference in the amount of antibody produced during continuous subculture for 3 months.

Example 4 2. Cell culture and purification of antibody:

The frozen cells of MP 5136 were thawed and cultured in 10% FCS medium on a large scale. After culturing the cells in NYSF 404 medium on a larger scale, the cells were suspended in 50 ml of NYSF 404 medium at a cell density of $5 \times 10^4$ /ml. The cells were then inoculated on 1 flask (bottom area of 175 cm$^2$) followed by stationary culture at 37$^\circ$C for 5 days in the presence of 5% carbon dioxide gas. The cells were collected and suspended in 500 ml of NYSF 404 medium at a cell density of $5 \times 10^4$/ml. The suspension was then inoculated on one starring culture flask (Techne) followed by spinner culture at 37$^\circ$C for 5 days at 20 rpm in the presence of 5% carbon dioxide gas. From 485 ml of the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 10.5 mg of IgM (N10-1) was obtained.

Example 5 3. Production of hybridoma capable of producing antibody to Pseudomonas aeruginosa:

(1) Preparation of EB virus solution:

EB virus solution was prepared in a manner similar to Example 3, (1).

(2) Preparation of human lymphocyte:

Heparinized peripheral blood, 50 ml, collected from healthy donors whose serum antibody activity to group B Pseudomonas aeruginosa was detected up to 1000-fold dilution by the DIBA method was used. Human lymphocytes were prepared in a manner similar to Example 3, (2) to give human lymphocyte pellets (cell number, $3.5 \times 10^7$).

(3) Transformation with EB virus:

Transformation with EB virus was performed in a manner similar to Example 3, (3), using $3.5 \times 10^7$ of the human antibody producing cells prepared in (2) and 35 ml of the virus solution prepared in (1). The transformant was inoculated on 672 wells in total. Three weeks after, cell growth was noted in all of the wells.

(4) Detection of antibody to Pseudomonas aeruginosa:

Group B serotype Pseudomonas aeruginosa ATCC 27578 was used as the formalinized dry bacteria. Anti Pseudomonas aeruginosa human antibody was detected in a manner similar to Example 1, (2).

(5) Cloning:

The cells in the 64 wells in which production of anti-group B Pseudomonas aeruginosa antibody was detected by the antibody detection method were transferred to a 24 well culture plate, a 6 well culture plate and a Petri dish of 6 cm in diameter, in this order, on an enlarged scale. With respect to the Petri dish in which cell growth was observed, the presence or absence of antibody to group B Pseudomonas aeruginosa in the culture supernatant was examined by the DIBA method in a manner similar to (4). Cloning was performed by the procedures similar to Example 3, (4), except for using the cells in the 16 Petri dishes showing a potent activity out of 52 dishes in which antibody production was detected. Three to five weeks after, the cells grew on soft agar and colonies were observed with the naked eye. Then, each colony was transferred to each well of a 96 well flat bottom culture plate, which had been previously charged separately with 0.1 ml of 20% FCS medium, using a Pasteur pipette. Two days after, 0.1 ml of 20% FCS medium was further added thereto and an additional 2 days thereafter, with respect to the wells where cell growth was observed, the presence or absence of human antibody to Pseudomonas aeruginosa in the culture supernatant was examined by the DIBA method. Antibody production was detected in soft agar colonies derived from 2 Petri dishes of 6 cm in diameter. These colonies were cultured sequentially on an enlarged scale to give EB virus transformed cell colonies 6L10N1, 6L10N2, 6L10N3 and 6L10N4 capable of producing human IgM antibody to group B Pseudomonas aeruginosa.

(6) Cell fusion:

Using $3 \times 10^7$ cells of EB virus transformed cell colony 6L10N2 capable of producing human IgM antibody to group B Pseudomonas aeruginosa and MP 4109, respectively, cell fusion was performed in a manner similar to Example 1. (1).

The fused cells were suspended in HA-O medium. After adjusting to a cell density of $5 \times 10^6$/ml, 0.1 ml each per well was inoculated on a 96 well flat bottom culture plate (192 wells in total). The cells were subjected to stationary culture at 37°C in the presence of 5% carbon dioxide gas. Four days after, 0.1 ml of HA-O medium was added thereto and thereafter, half of the volume of HA-0 medium was replaced by fresh HA-O medium at 3 to 5 day intervals. Four or 5 weeks after, cell growth was observed in 26 wells in total.

(7) Cloning:

The production of human antibody to group B Pseudomonas aeruginosa was detected in the culture supernatant of 20 wells among the culture supernatant of 26 wells in which cell growth was observed The cells of the 20 wells were cloned in a manner similar to Example 1, (3). Two to four weeks after, with respect to the wells where cell growth was observed, the presence or absence of human antibody to group B Pseudomonas aeruginosa in the culture supernatant was examined by the DIBA method. The cells of the wells in which production of antibody reactive with group B Pseudomonas aeruginosa was detected were again cloned in a manner similar to Example 1, (3). By cloning twice, 10 strains of MP hybridomas from MP 5090 to 5098 and MP 5147 capable of producing human monoclonal antibody reactive with group B Pseudomonas aeruginosa (ATCC 27588, ATCC 27583, ATCC 27589, IID 5004) were obtained.

MP 5097 was deposited in the Fermentation Research Institute of the Agency of Industrial Science and Technology of Japan under accession No. FERM BP-2268.

The hybridomas sufficiently grown in the 96 well flat bottom culture plate were cultured gradually on a larger scale. After the cells were suspended in a cell storage solution composed of 75% FCS, 10% DMSO and 15% RPMI 1640 medium at a density of $1 \times 10^6$ /ml, the suspension was separately charged in a 2 ml freezing tube. After cooling it to -20°C at a rate of 1°C/min, the tube was stored freezingly in liquid nitrogen.

(8) Determination of the amount of antibody produced:

The amount of antibody produced by 10 strains from MP 5090 to MP 5098 and MP 5147 was measured in a manner similar to Example 1, (4).

In the 10 hybridomas, $10^6$ cells secreted 8 to 38 $\mu$g of human IgM to the culture supernatant for 24 hours.

(9) Determination of stability of cell line in continuous subculture:

Stability of the growth and antibody production of MP 5093, MP 5095 and MP 5097 were examined in a manner similar to Example 1, (5).

Each of the three cell lines showed almost the same growth curve with the cells at the beginning of cultivation and 3 months after the cultivation. The doubling times of MP 5093, MP 5095 and MP 5097 calculated from the growth curves were 25.5, 25 and 25.5 hours, respectively. IgM secreted by $10^6$ cells for 24 hours was reduced in MP 5093 and MP 5095 during continuous subculture over 3 months. However, in MP 5097, IgM secreted by $10^6$ cells for 24 hours was 20 $\mu$g at the beginning of cultivation, 22 $\mu$g two months after and 15 $\mu$g 3 months after, indicating that there was no significant difference in the amount of antibody produced during continuous subculture for 3 months.

Example 6 3. Cell culture and purification of antibody:

The frozen cells of MP 5097 were thawed and cultured in 10% FCS medium on a large scale. After culturing the cells in NYSF 404 medium on a larger scale, the cells were suspended in 500 ml of NYSF 404 medium at a cell density of $5 \times 10^4$/ml. The suspension was then inoculated on ten flasks (bottom area of 175 cm$^2$) followed by stationary culture at 37°C for 5 days in the presence of 5% carbon dioxide gas. From the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 12.1 mg of IgM (N3-8) was obtained.

Example 7 4. Production of hybridoma capable of producing antibody to Pseudomonas aeruginosa :

(1) Cell fusion:

Using $3 \times 10^7$ of MP 4109 and MP 5038, respectively, cell fusion was carried out in a manner similar to Example 1, (1).

After the fused cells were suspended in HA-O medium at a cell density of $1 \times 10^5$/ml, the suspension was inoculated on a 96 well flat bottom culture plate in 0.1 ml each per well (576 wells in total). The cells were subjected to stationary culture at 37°C in the presence of 5% carbon dioxide gas.

Four days after, 0.1 ml of HA-O medium was added thereto and thereafter, half of the volume of HA-O medium was replaced by fresh HA-O medium at 3 to 5 day intervals. Four to five weeks after, cell growth was observed in 3 wells in total of 1E5, 3E2 and 4A11.

Using 5 x 10⁶ of MP 4109 and MP 5038, respectively, cell fusion was carried out as described above. After the fused cells were suspended in RPMI 1640 medium containing 20% FCS, 2 x 10⁻⁴ M of hypoxanthine, 0.66 $\mu$g/ml azaserine and 6 x 10⁻⁷ M of ouabain at a cell density of 1 x 10⁶/ml, the suspension was inoculated on a 96 well flat bottom culture plate in 0.1 ml each per well (96 wells in total). The cells were subjected to stationary culture at 37°C in the presence of 5% carbon dioxide gas. Four days after, 0.1 ml of RPMI 1640 medium containing 20% FCS, 2 x 10⁻⁴ M of hypoxanthine, 0.33 $\mu$g/ml azaserine and 3 x 10⁻⁷ M of ouabain (modified HA-O medium) was added thereto and thereafter, half of the volume of HA-O medium was replaced by fresh HA-O medium at 3 to 5 day intervals for the following 2 weeks. After two weeks passed, the medium was exchanged by RPMI 1640 medium containing 20% FCS and 2 x 10⁻⁴M of hypoxanthine. Four to five weeks after, cell growth was observed in 4 wells in total of 5A10, 5C3, 5C8 and 5G5.

(2) Detection of antibody to Pseudomonas aeruginosa:

Group E Pseudomonas aeruginosa ATCC 27581 was used as the formalinized dry bacteria. Anti-Pseudomonas aeruginosa antibody was detected in a manner similar to Example 1, (2).

(3) Cloning:

Among the culture supernatant of 7 wells in which cell growth was observed, the production of human antibody to group E Pseudomonas aeruginosa was detected in the culture supernatant of 5 wells of 1E5, 3E2, 4A11, 5C3 and 5G5. The cells of the 5 wells were cloned in a manner similar to Example 1, (3). Two to four weeks after, with respect to the wells where cell growth was observed, the presence or absence of human antibody to group E Pseudomonas aeruginosa in the culture supernatant was examined by the DIBA method. The cells of the wells in which production of antibody reactive with group E Pseudomonas aeruginosa was detected were again cloned in a manner similar to Example 1, (3). By cloning twice, 5 strains of hybridomas MP 5133, MP 5135, MP 5137, MP 5138 and MP 5139 capable of producing human monoclonal antibody which is cross-reactive with groups E and F Pseudomonas aeruginosa in the serologic classification by the Serotyping Comittee for the Japan Pseudomonas aeruginosa Society were obtained.

MP 5139 was deposited in the Fermentation Research Institute of the Agency of Industrial Science and Technology of Japan under accession No. FERM BP-2272.

The hybridomas sufficiently grown in a 96 well flat bottom culture plate were cultured gradually on a larger scale. After the cells were suspended in a cell storage solution composed of 75% FCS, 10% DMSO and 15% RPMI 1640 medium at a density of 1 x 10⁶ /ml, the suspension was separately charged in a 2 ml freezing tube. After cooling it to -20°C at a rate of 1°C/min, the tube was stored freezingly in liquid nitrogen.

(4) Determination of amount of antibody produced:

The amount of antibody produced by 5 strains of MP 5133, MP 5135, MP 5137, MP 5138 and MP 5139 was measured in a manner similar to Example 1, (4).

In hybridomas of MP 5133, MP 5135, MP 5137, MP 5138 and MP 5139, 10⁶ cells secreted 18 $\mu$g, 30 $\mu$g, 44 $\mu$g, 7 $\mu$g, and 45 $\mu$g of human IgM to the culture supernatant for 24 hours.

(5) Determination of stability of cell line in continuous subculture:

Stability of the growth and antibody production of MP 5139 were examined in a manner similar to Example 1, (5).

MP 5139 showed almost the same growth curve with the cells at the beginning of cultivation and 3 months after the cultivation. The doubling time calculated from the growth curve was 26 hours. Furthermore, IgM secreted by 10⁶ cells for 24 hours was 45 $\mu$g at the beginning of cultivation, 40 $\mu$g one month after the initiation of cultivation, 38 $\mu$g 2 months after and 36 $\mu$g 3 months after, indicating that there was no significant difference in the amount of antibody produced during continuous subculture for 3 months.

Example 8 4. Cell culture and purification of antibody:

The frozen cells of MP 5139 were thawed and cultured in 10% FCS medium on a large scale. After culturing the cells in NYSF 404 medium on a larger scale, the cells were suspended in 500 ml of NYSF 404 medium at a cell density of $5 \times 10^4$/ml. The suspension was then inoculated on ten flasks (bottom area of 175 cm²) followed by stationary culture at 37° C for 5 days in the presence of 5% carbon dioxide gas. From the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 10.6 mg of IgM (N1-5) was obtained.

Example 9 5. production of hybridoma capable of producing antibody to Pseudomonas aeruginosa:

(1) Preparation of EB virus solution:

EB virus solution was prepared in a manner similar to Example 3, (1).

(2) Preparation of human lymphocyte:

Heparinized peripheral blood, 50 ml, collected from healthy donor whose serum antibody activity to group E pseudomonas aeruginosa was detected up to 1000-fold dilution by the DIBA method was used. Human lymphocytes were prepared in a manner similar to Example 3, (2) to give human lymphocyte pellets (cell number, $5 \times 10^7$).

(3) Transformation with EB virus:

Transformation with EB virus was performed in a manner similar to Example 3, (3), using $5 \times 10^7$ of the human antibody producing cells prepared in (2) and 50 ml of the virus solution prepared in (1). With respect to the wells in which cell growth was observed, the presence or absence of antibody to group E pseudomonas aeruginosa in the culture supernatant was examined in a manner similar to Example 1, (2). The cells in the well in which antibody production was judged to be positive were cultured in a 24 well culture plate on a large scale.

(4) Cloning:

The cells in the wells in which production of human monoclonal antibody to group E pseudomonas aeruginosa antibody was detected by the antibody detection method were transferred to a petri dish of 6 cm in diameter. The cells grown in the Petri dish were cloned in a manner similar to Example 3, (4). Three to five weeks after, the cells grew on soft agar and colonies were observed with the naked eye. Then, each colony was transferred to each well of a 96 well flat bottom culture plate, which had been previously charged separately with 0.1 ml of 20% FCS medium, using a Pasteur pipette. Two days after, 0.1 ml of 20% FCS medium was also added and an additional 2 days after, with respect to the wells where cell growth was observed, the presence or absence of human antibody to Pseudomonas aeruginosa in the culture supernatant was examined by the DIBA method. The cells in the wells where antibody production was judged to be positive were cultured in a 24 well culture plate on an enlarged scale. Three days after, with respect to the wells in the 24 well culture plate, the presence or absence of human antibody to Pseudomonas aeruginosa in the culture supernatant was examined by the DIBA method. Among them, the cells in the wells in which the activity of human antibody to Pseudomonas aeruginosa was high in the culture supernatant were cultured in order on an enlarged scale to give EB virus transformed cell colony 86Z26AN6 capable of producing human IgM antibody to group E Pseudomonas aeruginosa.

(5) Cell fusion:

Using $2.5 \times 10^7$ each of EB virus transformed cell colony 86Z26AN6 capable of producing human IgM antibody to group E Pseudomonas aeruginosa and MP 4109, cell fusion was carried out in a manner similar to Example 1, (1). The fused cells were suspended in HA-O medium to adjust to a cell density of $1 \times 10^6$/ml, and 0.1 ml each per well was inoculated on a 96 well flat bottom culture plate (480 wells in total). The cells were subjected to stationary culture at 37° C in the presence of 5% carbon dioxide gas. Four days after, 0.1 ml of HA-O medium was added thereto and thereafter, half of the volume of HA-O medium was replaced by fresh HA-O medium at 4 or 5 day intervals. With respect to the 21 wells where cell grew, the presence or absence of human antibody to Pseudomonas aeruginosa in the culture supernatant was determined by the DIBA method. The antibody activity was detected in four wells of 1F2, 3A6, 3H11 and 4B4. And they were cloned, 3 strains of hybridomas MP 5140, MP 5141 and MP 5143 capable of producing human monoclonal antibody reactive only with group E Pseudomonas aeruginosa were obtained.

MP 5140 was deposited in the Fermentation Research Institute of the Agency of Industrial Science and Technology of Japan under accession No. FERM BP-2273.

(6) Determination of amount of antibody produced:

The amount of antibody produced by 3 strains of MP 5140, MP 5141 and MP 5143 was measured in a manner similar to Example 1, (4).

In hybridomas of MP 5140, MP 5141 and MP 5143, $10^6$ cells secreted 54 $\mu$g, 53 $\mu$g and 13 $\mu$g of human IgM in the culture supernatant for 24 hours, respectively.

(7) Determination of stability of cell line in continuous subculture:

Stability of the growth and antibody production of MP 5140 were examined in a manner similar to Example 1, (5).

MP 5140 showed almost the same growth curve with the cells at the beginning of cultivation and 3 months after the cultivation. The doubling time calculated from the growth curve was 24.5 hours. IgM secreted by $10^6$ cells for 24 hours was 54 $\mu$g at the beginning of cultivation, 45 $\mu$g one month after, 40 $\mu$g two months after and 38 $\mu$g three months after, indicating that there was no significant difference in the amount of antibody produced during continuous subculture for 3 months.

Example 10 5. Cell culture and purification of antibody:

The frozen cells of MP 5140 were thawed and cultured in 10% FCS medium on a large scale. After culturing the cells in NYSF 404 medium on a larger scale, the cells were suspended in 50 ml of NYSF 404 medium at a cell density of 5 x $10^4$/ml. The cells were then inoculated on 1 flask (bottom area of 175 cm²) followed by stationary culture at 37°C for 5 days in the presence of 5% carbon dioxide gas. The cells were collected and suspended in 500 ml of NYSF 404 medium at a cell density of 5 x $10^4$/ml. The suspension was then inoculated on one starring culture flask (Techne) followed by spinner culture at 37°C for 5 days at 20 rpm in the presence of 5% carbon dioxide gas. From 485 ml of the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 13.2 mg of IgM (NII-1) was obtained.

Example 11 6. Production of hybridoma capable of producing antibody to Pseudomonas aeruginosa:

(1) Cell fusion:

Using 1.5 x $10^7$ of MP 4109 and MP 5050, repectively, cell fusion was carried out in a manner similar to Example 1, (1).

After the fused cells were suspended in HA-O medium at a cell density of 1 x $10^6$/ml, the suspension was inoculated on a 96 well flat bottom culture plate in 0.1 ml each per well (288 wells in total). The cells were subjected to stationary culture at 37°C in the presence of 5% carbon dioxide gas. Four days after, 0.1 ml of HA-O medium was added thereto and thereafter, half of the volume of HA-O medium was replaced by fresh HA-O medium at 3 to 5 day intervals. Four to five weeks after, cell growth was observed in 10 wells in total.

(2) Detection of antibody to Pseudomonas aeruginosa:

As the formalinized dry bacteria, group G Pseudomonas aeruginosa ATCC 27584 was used. Anti-Pseudomonas aeruginosa human antibody was detected in a manner similar to Example 1, (2).

(3) Cloning:

Among the culture supernatant of the 10 wells in which cell growth was observed, the production of human antibody to group G Pseudomonas aeruginosa was detected in the culture supernatant of 8 wells. The cells of the 8 wells were cloned in a manner similar to Example 1, (3). Two to four weeks after, with respect to the wells where cell growth was observed, the presence or absence of human antibody to group G Pseudomonas aeruginosa in the culture supernatant was examined by the DIBA method. The cells of the wells in which production of antibody reactive with group 6 Pseudomonas aeruginosa was detected were again cloned in a manner similar to Example 1, (3). By cloning twice, 2 strains of hybridomas MP 5142 and MP 5151 capable of producing human monoclonal antibody which is cross-reactive with groups G and H Pseudomonas aeruginosa in the serologic classification by the Serotyping Comittee for the Japan Pseudomonas aeruginosa Society were obtained.

MP 5151 was deposited in the Fermentation Research Institute of the Agency of Industrial Science and Technology of Japan under accession No. FERM BP-2274.

The hybridomas sufficiently grown in a 96 well flat bottom culture plate were cultured gradually on a larger scale. After the cells were suspended in a cell storing solution composed of 75% FCS, 10% DMSO and 15% RPMI 1640 medium at a density of $1 \times 10^6$ /ml, the suspension was separately charged in a 2 ml freezing tube. After cooling to $-20\,^{\circ}$C at a rate of $1\,^{\circ}$C/min, the tube was stored freezingly in liquid nitrogen.

(4) Determination of amount of antibody produced:

The amount of antibody produced by 2 strains of MP 5142 and MP 5151 was measured in a manner similar to Example 1, (4).

In hybridomas of MP 5142 and MP 5151, $10^6$ cells secreted 16 $\mu$g and 18 $\mu$g of human IgM in the culture supernatant for 24 hours, respectively.

(5) Determination of stability of cell line in continuous subculture:

Stability of the growth and antibody production of MP 5151 were examined in a manner similar to Example 1, (5).

MP 5151 showed almost the same growth curve with the cells at the beginning of cultivation and 3 months after the cultivation. The doubling time calculated from the growth curve was 23.5 hours. Furthermore, IgM secreted by $10^6$ cells for 24 hours was 18 $\mu$g at the beginning of cultivation, 15 $\mu$g one month after the initiation of cultivation, 15 $\mu$g 2 months after and 13 $\mu$g 3 months after, indicating that there was no significant difference in the amount of antibody produced during continuous subculture for 3 months.

Example 12 6. Cell culture and purification of antibody:

The frozen cells of MP 5151 were thawed and cultured in 10% FCS medium on a large scale. After culturing the cells in NYSF 404 medium on a larger scale, the cells were suspended in 500 ml of NYSF 404 medium at a cell density of $5 \times 10^4$/ml. The suspension was then inoculated on ten flasks (bottom area of 175 cm²) followed by stationary culture at $37\,^{\circ}$C for 5 days in the presence of 5% carbon dioxide gas. From the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 13.7 mg of IgM (N7-2) was obtained.

Example 13 7. Production of hybridoma capable of producing antibody to Pseudomonas aeruginosa:

(1) Preparation of EB virus solution:

EB virus solution was prepared in a manner similar to Example 3, (1).

(2) Preparation of human lymphocyte:

Heparinized peripheral blood, 50 ml, collected from healthy donor whose serum antibody activity to group G Pseudomonas aeruginosa was detected up to 1000-fold dilution by the DIBA method was used. Human lymphocytes were prepared in a manner similar to Example 3, (2) to give human lymphocyte pellets (cell number, $4.8 \times 10^7$).

(3) Transformation with EB virus:

Transformation with EB virus was performed in a manner similar to Example 3, (3), using $4.8 \times 10^7$ of the human antibody producing cells prepared in (2) and 48 ml of the virus solution prepared in (1). The cells in the well in which antibody production was judged to be positive were cultured in a 24 well culture plate on a large scale.

(4) Cloning:

The cells in the wells in which production of human antibody to group G Pseudomonas aeruginosa antibody was detected by the antibody detection method were transferred to a Petri dish of 6 cm in diameter. The cells grown in the 6 cm Petri dish were cloned in a manner similar to Example 3, (4). Three to five weeks after, the cells grew on soft agar and colonies were observed with the naked eye. Then, each colony was transferred to each well of a 96 well flat bottom culture plate, which had been previously charged separately with 0.1 ml of 20% FCS medium, using a Pasteur pipette followed by stationary culture. Two days after, 0.1 ml of 20% FCS medium was further added thereto and an additional 2 days after, with respect to the wells where cell growth was observed, the presence or absence of human antibody to Pseudomonas aeruginosa in the culture supernatant was examined by the DIBA method. The cells in the wells wherein antibody production was judged to be positive were cultured in a 24 well culture plate on an enlarged scale. Three days after, with respect to the wells in the 24 well culture plate, the presence or absence of human antibody to Pseudomonas aeruginosa in the culture supernatant was examined by the DIBA method. Among them, the cells in the wells in which the activity of human antibody to Pseudomonas aeruginosa was high in the culture supernatant were cultured in order on an enlarged scale to give EB virus transformed cell colonies 7N19N1, 7N19N2, 7N19N3, 7N19N4, 7N19N5 and 7N19N6 capable of producing human IgM antibody to group G Pseudomonas aeruginosa.

(5) Cell fusion:

Using 1.0 x $10^7$ of a mixture of EB virus transformed cell colonies 7N19N1, 7N19N3, and 7N19N4 capable of producing human monoclonal IgM antibody to group G Pseudomonas aeruginosa and the same cell number of MP 4109, cell fusion was carried out in a manner similar to Example 1, (1). The fused cells were suspended in HA-O medium to adjust to a cell density of 1 x $10^6$/ml, and 0.1 ml each per well was inoculated on a 96 well flat bottom culture plate (192 wells in total). The cells were subjected to stationary culture at 37°C in the presence of 5% carbon dioxide gas. Four days after, 0.1 ml of HA-O medium was added thereto and thereafter, half of the volume of HA-O medium was replaced by fresh HA-O medium at 4 or 5 day intervals. With respect to the 37 wells where cells grew, the presence or absence of human antibody to Pseudomonas aeruginosa in the culture supernatant was determined by the DIBA method. The antibody activity was detected in the culture supernatant of the 37 wells. By cloning in a manner similar to Example 1, (3), 6 hybridomas of MP 5148, MP 5114, MP 5115, MP 5116, MP 5117 and MP 5118 capable of producing human monoclonal antibody reactive only with group G Pseudomonas aeruginosa were obtained.

MP 5114 was deposited in the Fermentation Research Institute of the Agency of Industrial Science and Technology of Japan under accession No. FERM BP-2259.

(6) Determination of amount of antibody produced :

An amount of antibody produced by 6 strains of MP 5148, MP 5114, MP 5115, MP 5116, MP 5117 and MP 5118 was measured in a manner similar to Example 1, (4).

In hybridomas of MP 5148, MP 5114, MP 5115, MP 5116, MP 5117 and MP 5118, $10^6$ cells secreted 10 μg, 18 μg, 10 μg, 12 μg, 11 μg and 15 μg of human IgM in the culture supernatant for 24 hours, respectively.

(7) Stability of cell line in continuous subculture:

Stability of the growth and antibody production of MP 5114 were examined in a manner similar to Example 1, (5).

MP 5114 showed almost the same growth curve with the cells at the beginning of cultivation and 3 months after the cultivation. The doubling time calculated from the growth curve was 24 hours. IgM secreted by $10^6$ cells for 24 hours was 18 μg at the beginning of cultivation, 12 μg one month after, 14 μg two months after and 15 μg three months after, indicating that there was no significant difference in the amount of antibody produced during continuous subculture for 3 months.

Example 14 7. Cell culture and purification of antibody:

The frozen cells of MP 5114 were thawed and cultured in 10% FCS medium on a large scale. After culturing the cells in NYSF 404 medium on a larger scale, the cells were suspended in 50 ml of NYSF 404 medium at a cell density of 5 x $10^4$ /ml. The cells were then inoculated on 1 flask (bottom area of 175 cm$^2$) followed by staticnary culture at 37°C for 5 days in the presence of 5% carbon dioxide gas. The cells were collected and suspended in 500 ml of NYSF 404 medium at a cell density of 5 x $10^4$/ml. The suspension was then inoculated on one starring culture flask (Techne) followed by spinner culture at 37°C for 5 days at 20 rpm in the presence of 5% carbon dioxide gas. From 485 ml of the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 11.9 mg of IgM (N8-2) was obtained.

Example 15 8. production of hybridoma capable of producing antibody to Pseudomonas aeruginosa:

(1) Cell fusion:

Using 4.5 x $10^7$ each of MP 4109 and MP 5035, respectively, cell fusion was carried out in a manner similar to Example 1, (1).

After the fused cells were suspended in HA-O medium at a cell density of 1 x $10^6$/ml, the suspension was inoculated on a 96 well flat bottom culture plate in 0.1 ml each per well (864 wells in total). The cells were subjected to stationary culture at 37°C in the presence of 5% carbon dioxide gas. Four days after, 0.1 ml of HA-O medium was added thereto and thereafter, half of the volume of HA-O medium was replaced by fresh HA-O medium at 3 to 5 day intervals. Four to five weeks after, cell growth was observed in 26 wells.

(2) Detection of antibody to Pseudomonas aeruginosa:

As the formalinized dry bacteria, group I Pseudomonas aeruginosa ATCC 27586 was used. Anti-Pseudomonas aeruginosa antibody was detected in a manner similar to Example 1, (2).

(3) Cloning:

Among the culture supernatant of 26 wells in which cell growth was observed, the production of human antibody to group I Pseudomonas aeruginosa was detected in the culture supernatant of 14 wells. The cells of the 14 wells were cloned in a manner similar to Example 1, (3). Two to four weeks after, with respect to the wells where cell growth was observed, the presence or absence of human antibody to group I Pseudomonas aeruginosa in the culture supernatant was examined by the DIBA method. The cells of the wells in which production of antibody reactive with group I Pseudomonas aeruginosa was detected were again cloned in a manner similar to Example 1, (3). By cloning twice, 2 strains of hybridomas MP 5156 and MP 5163 capable of producing human monoclonal antibody which is cross-reactive with groups I and D Pseudomonas aeruginosa in the serologic classification by the Serotyping Comittee for the Japan Pseudomonas aeruginosa Society were obtained.

MP 5156 was deposited in the Fermentation Research Institute of the Agency of Industrial Science and Technology of Japan under accession No. FERM BP-2339.

The hybridomas sufficiently grown in a 96 well flat bottom culture plate were cultured gradually on a larger scale. After the cells were suspended in a cell storing solution composed of 75% FCS, 10% DMSO and 15% RPMI 1640 medium at a density of 1 x $10^6$/ml, the suspension was separately charged in a 2 ml freezing tube. After cooling to -20°C at a rate of 1°C/min, the tube was stored freezingly in liquid nitrogen.

(4) Determination of amount of antibody produced:

The amount of antibody produced by 2 strains of MP 5156 and MP 5163 was measured in a manner similar to Example 1, (4).

In hybridomas of MP 5156 and MP 5163, $10^6$ cells secreted 15 μg and 10 μg of human IgM in the culture supernatant for 24 hours, respectively.

(5) Determination of stability of cell line in continuous subculture :

Stability of the growth and antibody production of MP 5156 were examined in a manner similar to Example 1, (5).

MP 5156 showed almost the same growth curve with the cells at the beginning of cultivation and 3 months after the cultivation. The doubling time calculated from the growth curve was 23.6 hours. Furthermore, IgM secreted by $10^6$ cells for 24 hours was 15 μg at the beginning of cultivation, 11 μg one month after the initiation of cultivation, 12 μg 2 months after and 10 μg 3 months after, indicating that there was no significant difference in the amount of antibody produced during continuous subculture for 3 months.

Example 16 8. Cell culture and purification of antibody :

The frozen cells of MP 5156 were thawed and cultured in 10% FCS medium on a large scale. After culturing the cells in NYSF 404 medium on a larger scale, the cells were suspended in 500 ml of NYSF 404 medium at a cell density of 5 x $10^4$/ml. The suspension was then inoculated on ten flasks (bottom area of 175 cm$^2$) followed by stationary culture at 37°C for 5 days in the presence of 5% carbon dioxide gas. From the culture, 480 ml of the supernatant was obtained by centrifugation (400 x g, 20 minutes) and filtered through a membrane filter having a pore size of 0.22 micron.

The antibody was purified from the filtrate in a manner similar to Example 2. From 480 ml of the culture supernatant, the solution containing 11.6 mg of IgM (N5-1) was obtained.

Example 17 1. Test on protective activity of human monoclonal antibody against Pseudomonas aeruginosa infectious diseases:

The protective activity of the human monoclonal antibodies N4-2 and N10-1 obtained in Examples 2 and 4 against group A Pseudomonas aeruginosa infections was examined. Five to 10 mice (Balb/c, female) of 8 to 12 weeks of age in one group were intraperitoneally administered 0.2 ml of each of solutions containing 50 ng, 500 ng, 5 $\mu$g and 50 $\mu$g of the human monoclonal antibody per mouse. Two hours after, the mice were challenged with a solution of group A Pseudomonas aeruginosa (F-1839) intraperitoneally. For the control group, physiological salt solution alone was administered instead of human monoclonal antibody. Pseudomonas aeruginosa was inoculated on heart infusion agar plate medium followed by culturing at 37°C overnight. The grown cell colonies were scraped out and diluted with physiological salt solution. To the dilution was added 5% mucin to prepare the bacterial solution in a challenge dose by 8.7 times the 50% lethal dose (LD$_{50}$ value) per mouse. After challenge with Pseudomonas aeruginosa, 50% effective dose (ED$_{50}$ value) was determined from the survival ratio of mice in each administration group on day 7. N4-2 and N10-1 showed ED$_{50}$ of 0.25 $\mu$g and 0.2 $\mu$g, respectively. Each human monoclonal antibody had a high protective activity against group A Pseudomonas aeruginosa infections.

Example 18 2. Test on protective activity of human monoclonal antibody against Pseudomonas aeruginosa infectious diseases:

The protective activity of the human monoclonal antibody N3-8 obtained in Example 6 against group B Pseudomonas aeruginosa infections was examined in a manner similar to Example 17. As the challenge bacteria, group B Pseudomonas aeruginosa (F-1860) was used and the bacterial amount was 20 times the LD$_{50}$ value. N3-8 showed ED$_{50}$ of 0.74 $\mu$g. The human monoclonal antibody had a high protective activity against group B Pseudomonas aeruginosa infections.

Example 19 3. Test on protective activity of human monoclonal antibody against Pseudomonas aeruginosa infectious diseases:

The protective activity of the human monoclonal antibodies NI-5 and NII-1 obtained in Examples 8 and 10 against group E Pseudomonas aeruginosa infections was examined in a manner similar to Example 17. As the challenge bacteria, group E Pseudomonas aeruginosa (PA 103) was used and the bacterial amount was 13.5 times the LD$_{50}$ value. NI-5 and NII-1 showed ED$_{50}$ of 0.135 $\mu$g and 0.39 $\mu$g, respectively. Each human monoclonal antibody had a high protective activity against group E Pseudomonas aeruginosa infec-

tions.

Example 20 4. Test on protective activity of human monoclonal antibody against Pseudomonas aeruginosa infectious diseases:

The protective activity of the human monoclonal antibodies N7-2 and N8-2 obtained in Examples 12 and 14 against group G Pseudomonas aeruginosa infections was examined in a manner similar to Example 17. As the challenge bacteria, group G Pseudomonas aeruginosa (P-28) was used and the bacterial amount was 8 times the LD$_{50}$ value. N7-2 and N8-2 showed ED$_{50}$ of 0.11 $\mu$g and 0.11 $\mu$g, respectively. Each human monoclonal antibody had a high protective activity against group G Pseudomonas aeruginosa infections.

Example 21 5. Test on protective activity of human monoclonal antibody against Pseudomonas aeruginosa infectious diseases:

The protective activity of the human monoclonal antibody N5-1 obtained in Example 16 against group I Pseudomonas aeruginosa infections was examined in a manner similar to Example 17. As the challenge bacteria, group I Pseudomonas aeruginosa (F-1856) was used and the bacterial amount was 11.0 times the LD$_{50}$ value. N5-1 showed ED$_{50}$ of 3.5 $\mu$g. The human monoclonal antibody had a high protective activity against group I Pseudomonas aeruginosa infections.

Example 22 1. Preparation of liquid composition:

N4-3 obtained in Example 2 and N10-1 obtained in Example 4 were, respectively, prepared in a concentration of 1 mg/ml with PBS(-) containing 0.2% (w/v)human serum albumin (Calbio). Each mixture was aseptically filtered through a membrane filter having a pore size of 0.22 micron. Each antibody solution was aseptically charged in vials, 1 ml/vial, to prepare each liquid composition. The compositions were allowed to stand at 4°C and 37°C for a month. Stability of the compositions during storage was determined by assaying antibody titer by ELISA using group A pseudomonas aeruginosa (ATCC 27577) LPS as antigen.

The antibody titer was determined as follows. LPS was dissolved (2 $\mu$g/ml) in 0.1 M citrate buffer (pH 4.0) and 0.05 ml each of the solution was separately charged in each well of a 96 well plate for EIA (Greiner Co.). After allowing it to stand at 37°C for 16 hours, LPS was adsorbed to the plate. Serial dilution of the liquid composition was reacted in the well at room temperature for 2 hours. Next, after reacting with peroxidase-conjugated goat anti-human IgM antibody (Tago) for 2 hours, a color was formed using as substrate 2,2'-azinobis(3-ethylbenzthiazolinesulfonic acid) (Sigma) and absorbance was measured at a wavelength of 414 nm. The dilution magnification showing absorbance of 0.1 was calculated by the method of least squares and this dilution value was made the antibody titer.

There was no difference in the antibody titers of N4-2 and N10-1 after allowing the solutions to stand between at 4°C or 37°C and at -80°C as a control for a month. Each antibody activity was maintained.

Example 23 2. Preparation of liquid composition:

A liquid composition of N3-8 obtained in Example 6 was prepared in a manner similar to Example 22. Stability of the liquid composition during storage was determined by assaying antibody titer by ELISA using group B Pseudomonas aeruginosa (ATCC 27578) LPS as antigen.

There was no difference in the antibody titer of N3-8 after allowing the solution to stand between at 4°C or 37°C and at -80°C as a control for a month. The antibody activity of N3-8 was maintained.

Example 24 3. Preparation of liquid composition:

Liquid compositions were prepared in a manner similar to Example 22 except for using NI-5 obtained in Example 8 and NII-1 obtained in Example 10. Stability of the liquid compositions during storage was determined by assaying antibody titer by ELISA using group E Pseudomonas aeruginosa (ATCC 27581) LPS as antigen.

There was no difference in the antibody titers of NI-5 and NII-1 after allowing them to stand between at 4°C or 37°C and at -80°C as a control for a month. Each antibody activity of the liquid compositions was maintained.

Example 25 4. Preparation of liquid composition:

Liquid compositions were prepared in a manner similar to Example 22 except for using N7-2 obtained in Example 12 and N8-2 obtained in Example 14. Stability of the liquid compositions during storage was determined by assaying antibody titer by ELISA using group G Pseudomonas aeruginosa (ATCC 27584) LPS as antigen.

There was no difference in the antibody titers of N7-2 and N8-2 after allowing them to stand between at 4°C or 37°C and at -80°C as a control for a month. Each antibody activity of the liquid compositions was maintained.

Example 26 5. Preparation of liquid composition:

Liquid composition of N5-1 obtained in Example 16 was prepared in a manner similar to Example 22. Stability of the liquid composition during storage was determined by assaying the antibody titer by ELISA using group I Pseudomonas aeruginosa (ATCC 27586) LPS as antigen.

There was no difference in the antibody titer of N5-1 after allowing it to stand between at 4°C or 37°C and at -80°C as a control for a month. The antibody activity of N5-1 was maintained.

Example 27 1. Preparation of lyophilized composition:

N4-2 obtained in Example 2 and N10-1 obtained in Example 4 were, respectively, prepared in a concentration of 1 mg/ml with PBS(-) containing 0.2% (w/v) human serum albumin (Cablio). Each mixture was aseptically filtered through a membrane filter having a pore size of 0.22 micron. Each antibody solution was aseptically charged in vials, lml/vial, and lyophilized to prepare each lyophilized composition. The lyophilized compositions, immediately after freeze drying and after allowing them to stand at 4°C and 37°C for 3 months and 6 months, were again dissolved in distilled water. The antibody titer was determined in a manner similar to Example 22 by ELISA using group A Pseudomonas aeruginosa (ATCC 27577) LPS as antigen. There was no difference in the antibody titers of N4-2 and N10-1 between the lyophilized compositions redissolved and the non-lyophilized compositions used for comparison. Each antibody activity was maintained.

Example 28 2. preparation of lyophilized composition:

Lyophilized composition of N3-8 obtained in Example 6 was prepared in a manner similar to Example 27. Stability of the lyophilized composition was determined by assaying the antibody titer by ELISA using group B Pseudomonas aeruginosa (ATCC 27578) LPS as antigen.

There was no difference in the antibody titer of the solution of N3-8 between the lyophilized composition redissolved and the non-lyophilized composition used for comparison. The antibody activity was maintained.

Example 29 3. Preparation of lyophilized composition:

Lyophilized compositions were prepared in a manner similar to Example 27 except for using N1-5 obtained in Example 8 and NII-1 obtained in Example 10. Stability of the lyophilized compositions was determined by assaying the antibody titer by ELISA using group E Pseudomonas aeruginosa (ATCC 27581) LPS as antigen.

There was no difference in the antibody titers of NI-5 and NII-1 between the lyophilized compositions redissolved and the non-lyophilized compositions used for comparison. Each antibody activity was maintained.

Example 30 4. Preparation of lyophilized composition:

Lyophilized compositions were prepared in a manner similar to Example 27 except for using N7-2 obtained in Example 12 and N8-2 obtained in Example 14. Stability of the lyophilized compositions was determined by assaying the antibody titer by ELISA using group G pseudomonas aeruginosa (ATCC 27584) LPS as antigen.

There was no difference in the antibody titers of the solutions of N7-2 and N8-2 between the lyophilized compositions redissolved and the non-lyophilized compositions used for comparison. Each antibody activity was maintained.

Example 31 5. Preparation of lyophilized composition:

Lyophilized composition of N5-1 obtained in Example 16 was prepared in a manner similar to Example 27. Stability of the lyophilized composition was determined by assaying the antibody titer by ELISA using group I Pseudomonas aeruginosa - (ATCC 27586) LPS as antigen.

There was no difference in the antibody titer of N5-1 between the lyophilized composition redissolved and the non-lyophilized composition used for for comparison. The antibody activity was maintanied.

EFFECTS OF THE INVENTION

The human monoclonal antibody, produced by culturing the human-human hybridoma of the present invention in a suitable medium for production and purified from the culture, is used singly or in a combination of two or more kinds of the human monoclonal antibody or in combination with other human antibodies, whereby excellent prophylactic and therapeutic effects against Pseudomonas aeruginosa infectious diseases can be exhibited.

The human-human hybridoma of the present invention is capable of producing a human monoclonal antibody reactive with at least one serological bacteria which are the major causative bacteria of Pseudomonas aeruginosa infectious diseases, the inventive human-human hybridoma can be continuously subcultured and grown stably in fetal calf serum-supplemented medium for animal tissue cul-ture over long periods cf time. In addition, the human-human hybridoma is also capable of producing the antibody in large quantities even in serum-free medium which is free of danger that the system might be contaminated by unknown impurities derived from the medium during the course of purifying the human monoclonal antibody from the culture. Thus, the human-human hybridoma is most suited for obtaining the human monoclonal antibody which can be raw materials for preparing compositions for prophylaxis and therapy of Pseudomonas aeruginosa infectious diseases. That is, the novel human-human hybridoma of the present invention has an enhanced ability of secreting the antibody can shorten the period for incubation, and thus can reduce production costs, when the human monoclonal antibody is produced by culturing on a large scale.

Furthermore, the human-human hybridoma of the present invention can be not only used as the cell line for producing human monoclonal antibody but also used as raw cells for preparing human antibody gene in the case of transfecting and expressing globulin gene in other host cells or microorganisms.

MICROORGANISMS DEPOSITED UNDER RULE 13.2

1. MP 4109

a. Depository
Name:      FERMENTATION RESEARCH IN-
                STITUTE, AGENCY OF INDUSTRI-
                AL SCIENCE AND TECHNOLOGY,
                MINISTRY OF INTERNATIONAL
                TRADE AND INDUSTRY
Address:   1-3, Higashi 1 chome, Tsukuba-shi,
                Ibaraki-ken 305, Japan
b. Deposition Date
October 27, 1988
c. Accession No. of Deposit

FERM BP-2129

2. MP 5046

a. Depository
Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Deposition Date
December 9, 1987
c. Accession No. of Deposit
FERM BP-1599

3. MP 5038

a. Depository
Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Deposition Date
December 7, 1987
c. Accession No. of Deposit
FERM BP-1596

4. MP 5050

a. Name:        FERMENTATION RESEARCH Institute, AGENCY OF INDUSTRIAL
SCIENCE AND TECHNOLOGY,
MINISTRY OF INTERNATIONAL
TRADE AND INDUSTRY
b. Deposition Date
December 9, 1987
c. Accession No. of Deposit
FERM BP-1600

5. MP 5035

a. Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
b. Deposition Date
December 9, 1987
c. Accession No. of Deposit
FERM BP-1598

6. MP 5121

a. Name        : FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Deposition Date
February 7, 1989
c. Accession No. of Deposit
FERM BP-2270

7. MP 5136

a. Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Deposition Date
February 7, 1989
c. Accession No. of Deposit
FERM BP-2271

8. MP 5097

a. Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Deposition Date
February 7, 1989
c. Accession No. of Deposit
FERM BP-2268

9. MP 5139

a. Name:        FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address:        1-3, Higashi 1 chome, Tsukuba-shi,
Ibaraki-ken 305, Japan
b. Depositon Date
February 7, 1989
c. Accession No. of Deposit
FERM BP-2272

10. MP 5140

a. Name: FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
B. Deposition Date
February 7, 1989
c. Accession No. of Deposit
FERM BP-2273

11. MP 5114

a. Name: FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address: 1-3, Higashi 1 chome, Tsukuba-shi,
Ibaraki-ken 305, Japan
b. Deposition Date
February 7, 1989
c. Accession No. of Deposit
FERM BP-2269

12. MP 5151

a. Name: FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address: 1-3, Higashi 1 chome, Tsukuba-shi,
Ibaraki-ken 305, Japan
b. Deposition Date
February 7, 1989
c. Accession No. of Deposit
FERM BP-2274

13. MP 5156

a. Name: FERMENTATION RESEARCH IN-
STITUTE, AGENCY OF INDUSTRI-
AL SCIENCE AND TECHNOLOGY,
MINISTRY OF INTERNATIONAL
TRADE AND INDUSTRY
Address: 1-3, Higashi 1 chome, Tsukuba-shi,
Ibaraki-ken 305, Japan
b. Deposition Date
March 16, 1989
c. Accession No. of Deposit
FERM BP-2339

## Claims

1. Human-human hybridoma capable of producing human monoclonal antibody having reactivity with at least one serotype bacteria which
are major causative bacteria of Pseudomonas
aeruginosa infections and a cell line derived
therefrom.

2. Human-human hybridoma and a cell line derived therefrom according to claim 1, wherein
said human monoclonal antibody has reactivity
at least with group A Pseudomonas
aeruginosa.

3. Human-human hybridoma and a cell line derived therefrom according to claim 1, wherein
said human monoclonal antibody has reactivity
at least with group B Pseudomonas
aeruginosa.

4. Human-human hybridoma and a cell line derived therefrom according to claim 1, wherein
said human monoclonal antibody has reactivity
at least with group E Pseudomonas
aeruginosa.

5. Human-human hybridoma and a cell line derived therefrom according to claim 1, wherein
said human monoclonal antibody has reactivity
at least with group G Pseudomonas
aeruginosa.

6. Human-human hybridoma and a cell line derived therefrom according to claim 1, wherein
said human monoclonal antibody has reactivity
at least with group 1 Pseudomonas
aeruginosa.

7. Human-human hybridoma and a cell line derived therefrom according to any one of claims
1 through 6, which is a hybridoma between a
cell line having a human chromosome alone
and capable of infinite growth and human antibody producing cells.

8. Human-human hybridoma and a cell line derived therefrom according to claim 7, wherein
said cell line having a human chromosome
alone and capable of infinite growth is a cell
line having the selective characteristics of a
hybridoma.

9. Human-human hybridoma and a cell line derived therefrom according to claim 8, wherein
said cell line having the selective characteristics of hybridoma is FERM BP-2129 or a cell
line derived therefrom.

10. Human-human hybridoma and a cell line derived therefrom according to claim 7, wherein
said human antibody producing cells are EB
virus transformed cells.

11. Human-human hybridoma and a cell line derived therefrom according to claim 10, wherein said EB virus transformed cells are FERM BP-1599, 1596, 1600, 1598 or a cell line derived therefrom.

12. Human-human hybridoma and a cell line derived therefrom according to any one of claims 1 through 11, wherein said human monoclonal antibody produced is protective against infections with the corresponding serotype Pseudomonas aeruginosa.

13. Human-human hybridoma of FERM BP-2270, 2271, 2268, 2272, 2273, 2269, 2274, 2239 or a cell line derived therefrom.

14. A process for producing human-human hybridoma according to any one of claims 1 through 13.

15. A process for producing human monoclonal antibody which comprises culturing at least one human-human hybridoma according to any one of claims 1 through 13 and a cell line derived therefrom, and purifying human monoclonal antibody from the culture.

16. A human monoclonal antibody produced by the human-human hybridoma according to any one of claims 1 through 13 and a cell line derived therefrom.

17. A human monoclonal antibody prepared by the process of claim 15.

18. A pharmaceutical composition for the prophylaxis or therapy of Pseudomonas aeruginosa infectious diseases comprising at least one human monoclonal antibody according to claims 11 or 12.

19. A pharmaceutical composition for the prophylaxis or therapy of Pseudomonas aeruginosa infectious diseases according to claim 18, which is a liquid composition.

20. A pharmaceutical composition for the prophylaxis or therapy of Pseudomonas aeruginosa infectious diseases according to claim 18, which is a lyophilized composition.

21. A method for the prophylaxis or therapy of Pseudomonas aeruginosa infectious diseases which comprises administering a composition for the prophylaxis or therapy of Pseudomonas aeruginosa infectious diseases according to claims 18 through 20.

**I. CLASSIFICATION OF SUBJECT MATTER** (if several classification symbols apply, indicate all) [6]

According to International Patent Classification (IPC) or to both National Classification and IPC

Int. Cl$^5$     C12N5/24, C12P21/08, A61K39/395

**II. FIELDS SEARCHED**

| Minimum Documentation Searched [7] | |
|---|---|
| Classification System | | Classification Symbols |
| IPC | C12N5/00, C12N21/00 |

Documentation Searched other than Minimum Documentation
to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| P | JP, A, 2-429 (Takeda Chemical Industries, Ltd.), 5 January 1990 (05. 01. 90) & EP, A, 322762 | 1 - 21 |
| P | JP, A, 1-197500 (Sumitomo Chemical Co., Ltd. and another), 9 August 1989 (09. 08. 89) & EP, A, 326148 & GB, A, 2214509 & FR, A, 2626473 | 1 - 21 |
| Y | JP, A, 60-248626 (Mitsui Toatsu Chemicals, Inc.), 9 December 1985 (09. 12. 85), (Family: none) | 1 - 21 |
| Y | JP, A, 60-203186 (The Green Cross Corporation), 14 October 1985 (14. 10. 85) & EP, A, 157574 | 1 - 21 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 2, 1990 (02. 06. 90) | June 18, 1990 (18. 06. 90) |

| International Searching Authority | Signature of Authorized Officer |
|---|---|
| Japanese Patent Office | |

Form PCT/ISA/210 (second sheet) (January 1985)

| FURTHER INFORMATION CONTINUED FROM THE SECOND SHEET | | |
|---|---|---|
| Y | JP, A, 62-187417 (Genetic Systems Co.), 15 August 1987 (15. 08. 87) & DE, A, 3642095 & GB, A, 2185266, & FR 2593826 | 1 - 21 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers          , because they relate to subject matter not required to be searched by this Authority, namely:

2.☐ Claim numbers          , because they relate to parts of the international application that do not comply with the prescribed requirements to such an extent that no meaningful international search can be carried out, specifically:

3.☐ Claim numbers ..          , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)